(19) Europäisches Patentamt
European Patent Office
Office européen des brevets

(11) **EP 4 368 251 A2**

(12) **EUROPEAN PATENT APPLICATION**

(43) Date of publication:
**15.05.2024 Bulletin 2024/20**

(21) Application number: **24152887.6**

(22) Date of filing: **21.08.2020**

(51) International Patent Classification (IPC):
***A61P 35/02*** (2006.01)

(52) Cooperative Patent Classification (CPC):
**A61K 38/50; A61K 47/60;** Y02A 50/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.08.2019 GB 201912020**

(62) Document number(s) of the earlier application(s) in accordance with Art. 76 EPC:
**20765071.4 / 4 017 525**

(71) Applicant: **Porton Biopharma Limited Salisbury, Wiltshire SP4 0JG (GB)**

(72) Inventor: **GERVAIS, David Salisbury, SP4 0JG (GB)**

(74) Representative: **Titmus, Craig Edward Mathys & Squire The Shard 32 London Bridge Street London SE1 9SG (GB)**

Remarks:
•The complete document including Reference Table(s) and the Sequence Listing(s) can be downloaded from the EPO website
•This application was filed on 19-01-2024 as a divisional application to the application mentioned under INID code 62.
•Claims filed after the date of filing/after the date of receipt of the divisional application (Rule 68(4)EPC).

(54) **THERAPEUTIC CONJUGATE**

(57)    The invention provides conjugates comprising L-asparaginase and a water-soluble polymer for use in treating a disease treatable by L-asparagine depletion in a patient that has been previously administered E. coli derived L-asparaginase. The invention also provides methods of treatment, compositions comprising said conjugate, and methods of producing the conjugate.

Figure 1

EP 4 368 251 A2

**Description**

The present invention relates to conjugates comprising L-asparaginase and a water-soluble polymer for use in treating a disease treatable by L-asparagine depletion in a patient that has been previously administered *E. coli* derived L-asparaginase. The present invention also relates to compositions comprising said conjugate, and to methods of producing the conjugate.

For many years, L-asparaginases have successfully been used in the treatment of cancers that are dependent upon extracellular L-asparagine. L-asparaginases catalyse the hydrolysis of L-asparagine to aspartic acid and ammonia, and it is believed that their antineoplastic activity is achieved by depleting circulating L-asparagine levels, thereby killing tumor cells which rely upon extracellular L-asparagine for protein synthesis.

L-asparaginases are an essential component in the treatment of Acute Lymphoblastic Leukaemia (ALL), and have also been used to treat cancers such as acute myelocytic leukaemia, Hodgkin's disease, acute myelomonocytic leukaemia, chronic lymphocytic leukaemia, reticulosarcoma, melanosarcoma and lymphosarcoma.

To date, three bacterial-derived L-asparaginase preparations have been approved for use in the treatment of ALL. These include native *E.coli* L-asparaginase, PEGylated *E. coli* L-asparaginase and native *Erwinia chrysanthemi* asparaginase.

Native *E.coli* L-asparaginase ("EcASNase") has been marketed as "L-Asparaginase Medac®" and "Kidrolase®" in Europe, and as "Elspar®" in the USA. During the 1970s, EcASNase was identified as an effective drug for the treatment of ALL, and its administration leads to rapid and strong depletion of L-asparagine. However, many patients treated with EcASNase displayed undesirable "overt" allergic symptoms, and anti-L-asparaginase antibody responses were observed in most patients. Anti-L-asparaginase antibodies can have highly deleterious therapeutic effects because they can block the enzymatic activity of L-asparagine and increase the rate of L-asparagine clearance from the patient. Native *E.coli* L-asparaginase is no longer approved for clinical use in the USA.

To help reduce immunological and pharmacokinetic problems associated with EcASNase, EcASNase was conjugated to polyethylene glycol ("PEG"). PEG is a well-known water-soluble polymer, and conjugation to PEG is a long-established strategy to improve pharmacokinetic and immunological properties of proteins. Well-known advantages of PEGylation include improved residual enzymatic activity, improved thermal stability, improved pH stability, increased resistance to proteolysis, increased *in vivo* half-life ($t_{1/2}$), and reduced antigenicity. Consistent with the well-known advantages of PEGylation, PEGylated EcASNase displayed *inter alia* reduced antigenicity and increased $t_{1/2}$, as compared to native EcASNase. Since 2006, PEGylated EcASNase has been approved for first-line treatment of ALL in children and adults. PEGylated EcASNase is frequently referred to in the literature as "pegaspargase" and is marketed as "Oncaspar®". Oncaspar® is *E. coli* L-asparaginase purified from *E. coli* and modified at multiple sites with 5000 Da PEG.

For the treatment of ALL, Oncaspar® is typically administered every 14 days, as a 750 IU (International Units)/ml solution for injection/infusion. In paediatric patients aged ≤21 years, the recommended dose is typically: (a) 2,500 IU Oncaspar® (equivalent to 3.3 ml Oncaspar®)/m² body surface area in patients with a body surface area (BSA) ≥0.6 m²; and (b) 82.5 IU of Oncaspar® (equivalent to 0.1 ml Oncaspar)/kg body weight in patients with a BSA of <0.6 m². In patients aged >21 years (unless prescribed otherwise) Oncaspar® is typically administered at a dose of 2,000 IU (equivalent to 2.67 ml Oncaspar)/m² body surface area every 14 days.

Despite its improvements over EcASNase, Oncaspar® is hampered by significant limitations. In particular, administration of Oncaspar® is frequently associated with overt allergic symptoms, particularly in patients who have previously received Oncaspar® or EcASNase. Also of major clinical concern is the phenomenon of "silent inactivation", whereby patients develop an anti-L-asparaginase antibody response to Oncaspar® (or EcASNase), but do not display overt allergic symptoms. A major concern with silent inactivation is that patients are at risk of receiving continued administration of Oncaspar® (or EcASNase) without receiving the therapeutic benefit.

Native *E. chrysanthemi* asparaginase (EwASNase) is approved for second- or third-line treatment of ALL in patients who have developed hypersensitivity to *E. coli* L-asparaginase (typically Oncaspar®). EwASNase that has been purified from *E. chrysanthemi* strain NCPPB 1066 is marketed as "Erwinaze®" in the USA and "Erwinase®" elsewhere. EwASNase has minimal antigenic cross-reactivity with EcASNase (in native or PEGylated forms), and so patients who have previously received Oncaspar® (or EcASNase) are not immunologically primed to respond to EwASNase. Thus, EwASNase is well-suited to the continued treatment of patients who have developed a hypersensitive reaction to Oncaspar® (or EcASNase), as well as patients who have developed an anti-L-asparaginase antibody response to Oncaspar® (or EcASNase).

Erwinase® is typically provided as 10,000 IU/vial lyophilisate for solution for injection. For all patients the usual Erwinase® dose is 6,000 IU/m² body surface area (200 IU/kg of body weight). Erwinase®-based therapy may be further intensified according to protocol.

Despite its immunological advantages, EwASNase displays a shorter $t_{1/2}$ than Oncaspar® (and EcASNase). To help match the *in vivo* L-asparaginase activity provided over time by Oncaspar® (and EcASNase) patients receive shorter administration intervals of EwASNase. For the treatment of ALL, Erwinase® is typically administered three times per week for three weeks.

There exists a considerable desire in the art to extend the administration intervals of EwASNase. This desire is recognised by Rau et al. Pediatr Blood Cancer. 2018; 65:e26873, which notes that: *"Erwinia asparaginase has proven to be a safe and effective alternative to E. coli PEG-asparaginase* [Oncaspar®]*"*, and that *"reduced frequency-dosing regimen would provide important benefits"*.

Rau *et al.* (2018) also notes that: *"Because it is a large, bacteria-derived protein, exposure to asparaginase has the capacity to elicit an immune response"*, and that *"preparations with reduced immunogenic potential would provide important therapeutic benefits"*.

Rau *et al.* (2018) describes the results of Children's Oncology Group trial AALL1421. This was a phase 2 clinical trial of "JZP-416" in pediatric patients with ALL or lymphoblastic lymphoma and hypersensitivity to Oncaspar®.

"JZP-416" is a conjugate of recombinant *E. chrysanthemi* L-asparaginase (expressed in *E. coli*) and 5000 Da PEG. At present, "JZP-416" is also referred to in the art as "Asparec®", "AZP-02" and "mPEG-r-crisantaspase", and is owned by Jazz Pharmaceuticals (previously Alize Pharma II SAS).

Unfortunately, Children's Oncology Group trial AALL1421 was unsuccessful because three of the four patients *"experienced hypersensitivity to* [JZP-416] *manifested as clinical hypersensitivity reactions or rapid clearance of* [serum asparaginase activity]*"* (Rau *et al.* (2018)).

Failure of trial AALL1421 was unexpected because:

(a) L-asparaginase purified from *E. chrysanthemi* strain NCPPB 1066 (Erwinase®) is approved for use with patients who have developed hypersensitivity to Oncaspar®; and

(b) PEG *per se* is generally considered to be non-immunogenic. Indeed, PEGylation is a well-known strategy to *reduce* immunogenicity of proteins (see above).

In an attempt to explain the failure of trial AALL1421, Rau *et al.* (2018) comments that there should be *"no cross-reactivity between the L-asparaginase portion of pegcrisantaspase* [JZP-416] *and pegaspargase* [Oncaspar®]*"* on the basis that *E. coli* L-asparaginase (in Oncaspar®) has a different amino acid sequence from the amino acid sequence of *E. chrysanthemi* L-asparaginase (in JZP-416).

Instead, Rau *et al.* (2018) attributes the failure of Children's Oncology Group trial AALL1421 to *"pre-existing immunogenicity against the PEG moiety of* [JZP-416]*"*, and their conclusion was supported by the identification of pre-existing anti-PEG IgG antibodies in AALL1421 trial patients.

Ultimately, Rau *et al.* (2018) concludes that *"strategies other than PEGylation may be needed to optimize the Erwinia asparaginase treatment schedule for pegaspargase-hypersensitive patients"*.

Thus, there exists a need in the art for an L-asparaginase which has longer administration intervals than Erwinase®, and is suitable for the treatment of patients who have developed a hypersensitive reaction to Oncaspar® (or EcASNase).

There also exists a need in the art for an L-asparaginase which has longer administration intervals than Erwinase®, and is suitable for the treatment of patients who have developed an anti-L-asparaginase antibody response to Oncaspar® (or EcASNase), but do not display overt allergic symptoms.

The present invention addresses the need for an L-asparaginase which has longer administration intervals than Erwinase®, and is suitable for the treatment of patients who have developed a hypersensitive reaction to Oncaspar® (or EcASNase). The present invention also addresses the need for an L-asparaginase which has longer administration intervals than Erwinase®, and is suitable for the treatment of patients who have developed an anti-L-asparaginase antibody response to Oncaspar® (or EcASNase), but do not display overt allergic symptoms.

The present invention is based on the surprising discovery that the failure of Children's Oncology Group trial AALL1421 was not due to *"pre-existing immunogenicity against the PEG moiety of* [JZP-416]*"*, as was concluded by Rau *et al.* (2018). Instead, the inventors have unexpectedly discovered that failure of trial AALL1421 was due to pre-existing immunity to host cell proteins (HCPs) from *E. coli,* which were present in Oncaspar® and also present in JZP-416. Put another way, the inventors believe that previous administration of Oncaspar® had immunologically "primed" the patients to elicit a hypersensitive immune response to *E. coli* HCPs that were present in the *E. coli*-derived L-asparaginase preparation, JZP-416.

Host cell proteins (HCPs) are endogenous proteins expressed by host cells and are unrelated to the therapeutic products produced by said host cells. HCPs constitute a major component of process-related impurities in biologic drugs, and it has been reported that HCPs often are co-purified with the therapeutic product by interacting with the therapeutic product itself.

Thus, the invention provides a conjugate comprising L-asparaginase and a water-soluble polymer, for use in treating a disease treatable by L-asparagine depletion in a patient, wherein:

(a) the L-asparaginase is from a source other than *E. coli;*

(b) the L-asparaginase is expressed in a host cell other than *E. coli;* and

(c) the patient has previously been administered *E. coli*-derived L-asparaginase.

The invention also provides a recombinant heterologously-expressed L-asparaginase for use in treating a disease treatable by L-asparagine depletion in a patient, wherein:

(a) the L-asparaginase is from a source other than *E. coli;*

(b) the heterologous host cell is a host cell other than *E. coli;* and

(c) the patient has previously been administered *E. coli*-derived L-asparaginase.

According to the invention, the *E. coli*-derived L-asparaginase previously administered to the patient is typically Oncaspar®.

Typically, the L-asparaginase is recombinantly expressed in the host cell (*i.e.* the L-asparaginase is a *"recombinant* L-asparaginase").

In one embodiment, the host cell is a heterologous host cell.

In one embodiment, the L-asparaginase is from a source other than *E. coli* and purified from a host cell other than *E. coli* (*e.g.* native *E. chrysanthemi* L-asparaginase purified from *E. chrysanthemi*).

The invention also provides a composition comprising the conjugate for use according to the invention and a pharmaceutically acceptable excipient.

In one embodiment, the conjugate of the invention comprises recombinant heterologously-expressed L-asparaginase (*e.g. E. chrysanthemi* L-asparaginase expressed in *Pseudomonas spp.*).

The invention also provides a composition comprising the recombinant heterologously-expressed L-asparaginase for use according to the invention and a pharmaceutically acceptable excipient.

Typically, the composition of the invention is substantially free from host cell proteins from *E. coli.* Preferably, the composition of the invention does not contain host cell proteins from *E. coli.*

The invention also provides a method of treating a disease treatable by L-asparagine depletion in a patient that has been previously administered *E. coli*-derived L-asparaginase, said method comprising administering to said patient an effective amount of the conjugate or composition of the invention.

The present invention provides significant and real-world therapeutic advantages:

•     The present invention significantly reduces the risk of (further) hypersensitive reactions in patients that were previously treated with Oncaspar® (or EcASNase);

•     The present invention provides the skilled person with increased freedom of choice when selecting water-soluble polymer(s) for use in L-asparaginase conjugates of the invention. For example, the skilled person may enjoy the well-known immunological and pharmacokinetic advantages provided by PEGylation, as well as other types of water-soluble polymer;

•     Critically, having identified the key antigenic determinant responsible for the failure of Children's Oncology Group trial AALL1421, disclosure of the present invention will also help avoid future administration of *E. coli*-derived L-asparaginase to cancer patients who have developed sensitivity to Oncaspar® (or EcASNase). This will, in turn, help avoid further patient suffering in an already vulnerable patient group.

The inventors have determined for the first time that patients who have previously been administered *E. coli*-derived L-asparaginase (such as Oncaspar®) are immunologically pre-disposed to elicit a hypersensitive response to L-asparaginase that is derived from *E. coli.*

Moreover, patients who have previously been administered *E. coli*-derived L-asparaginase (such as Oncaspar®) are immunologically pre-disposed to elicit a hypersensitive response to L-asparaginase that is derived from *E. coli,* irrespective of whether the subsequently-administered *E. coli*-derived L-asparaginase is conjugated to another moiety (such as PEG).

As used herein, the term *"E. coli-derived L-asparaginase"* refers to L-asparaginase that was produced in *E. coli* (either by recombinant or endogenous expression). Thus, according to the invention, the term *"E. coli*-derived L-asparaginase" includes:

(a) Native *E. coli* L-asparaginase purified from *E. coli;*

(b) Recombinant L-asparaginase <u>expressed</u> in *E. coli,* irrespective of the source or amino acid sequence of L-asparaginase (*i.e.* irrespective of whether the recombinantly expressed L-asparaginase is *E. coli* L-asparaginase, or whether the recombinantly expressed L-asparaginase is from a source other than *E. coli,* such as from *E. chrysanthemi*); and

(c) compositions and/or conjugates comprising (a) or (b), above.

The *E. chrysanthemi* L-asparaginase component of JZP-416 was recombinantly expressed *in* E. *coli.* Thus, JZP-416 is an *"E. coli*-derived L-asparaginase" according to the present invention.

As used herein, the term *"L-asparaginase from a source other than E. coli"* refers to L-asparaginases (either conjugated or un-conjugated) that have an amino acid sequence that is different from the amino acid sequence of *E. coli* L-asparaginase. *"E. coli asparaginases"* include EcASNase and Oncaspar®, and the amino acid sequence of SEQ ID NO: 1. Typically, an L-asparaginase from a source other than *E. coli* has less than 90% sequence identity to the amino acid sequence of SEQ ID NO: 1 (*e.g.* less than 90%, less than 85%, less than 80%, less than 75% sequence identity to the amino acid sequence of SEQ ID NO: 1).

MEFFKKTALAALVMGFSGAALALPNITILATGGTIAGGGDSATKSNYTVGKVGVENL

VNAVPQLKDIANVKGEQVVNIGSQDMNDNVWLTLAKKINTDCDKTDGFVITHGTDT

MEETAYFLDLTVKCDKPVVMVGAMRPSTSMSADGPFNLYNAVVTAADKASANRGV

LVVMNDTVLDGRDVTKTNTTDVATFKSVNYGPLGYIHNGKIDYQRTPARKHTSDTP

FDVSKLNELPKVGIVYNYANASDLPAKALVDAGYDGIVSAGVGNGNLYKSVFDTLAT

AAKTGTAVVRSSRVPTGATTQDAEVDDAKYGFVASGTLNPQKARVLLQLALTQTK

DPQQIQQIFNQY **(SEQ ID NO: 1)**

Thus, according to the invention, EwASNase and JZP-416 are L-asparaginases from a source other than *E. coli.* The amino acid sequence of EwASNase (and JZP-416) is provided by SEQ ID NO: 2.

ADKLPNIVILATGGTIAGSAATGTQTTGYKAGALGVDTLINAVPEVKKLANVKGEQFS

NMASENMTGDVVLKLSQRVNELLARDDVDGVVITHGTDTVEESAYFLHLTVKSDKP

VVFVAAMRPATAISADGPMNLLEAVRVAGDKQSRGRGVMVVLNDRIGSARYITKTN

ASTLDTFKANEEGYLGVIIGNRIYYQNRIDKLHTTRSVFDVRGLTSLPKVDILYGYQD

DPEYLYDAAIQHGVKGIVYAGMGAGSVSVRGIAGMRKAMEKGVVVIRSTRTGNGIV

PPDEELPGLVSDSLNPAHARILLMLALTRTSDPKVIQEYFHTY **(SEQ ID NO: 2)**

The skilled person will appreciate that the term *"expressed in a host cell other than E. coli"* embraces endogenous expression and recombinant expression, as appropriate. L-asparaginases are enzymes having L-asparagine aminohydrolase activity.

One International Unit ("IU") of L-asparaginase activity is defined as the amount of enzyme that catalyses the release of 1 μmol of ammonia per minute at 37°C. Serum L-asparaginase activity (SAA) levels of ≥0.1 IU/ml are generally considered to provide therapeutic reduction of asparagine.

Numerous L-asparaginases have been identified in a variety of different source organisms such as bacteria, plants and fungi. As noted above, *E. coli* L-asparaginase and *E. chrysanthemi* L-asparaginase are the only L-asparaginases that have been clinically approved for the treatment of ALL.

Sequence information on L-asparaginases is readily available via online databases, such as https://www.ncbi.nlm.nih.gov/. L-asparaginase from *E. chrysanthemi* is ideally-suited to use in the invention. Reference *E. chrysanthemi* L-asparaginase amino acid sequence is provided by SEQ ID NO: 2.

In one embodiment, the L-asparaginase has at least 80% identity to the amino acid sequence of SEQ ID NO:

2, *e.g.* at least 81, 82, 83, 84, 85, 86, 87, 88, 89, 90, 91, 92, 93, 94, 95, 96, 97, 98, 99 or 100% identity to the amino acid sequence of SEQ ID NO: 2.

The inventors believe that L-asparaginase from sources other than *E. chrysanthemi* (except for *E. coli*-derived L-asparaginase) would also provide the advantageous technical effects which characterise the invention.

For example, in one embodiment, the L-asparaginase is *Pseudomonas putida.* L-asparaginase. A reference *Pseudomonas putida* L-asparaginase sequence is provided by SEQ ID NO: 3:

MIDHSSLPRLSIASLGGTVSMQAQAVGCGVTPTLDCEQQLLQVPQLRQMAQLNVA

SLCLVPSASLDFATLLDVLAWARCEVERGAQALVVSQGTDSLEESAYFLDLLWPFD

APLVMTGAMRSASQPGNDGPANLLAAAQVALAQGSCGRGVLVVMNDQVHRAAR

VRKTASMAIAAFESPGCGPLGEVVEGKVVYRHPPARGEVLPVPHRTDQRVALLEA

CLDADTALLQAVAPLGYEGLVIAGFGAGHVAASWSDVLEQLAPTLPVVVATRTGNG

PTARATYGFAGAEIDLQKKGVYMAGHLCPRKCRILLWLLIGTDRRHELHDWLHA

**(SEQ ID NO: 3)**

In one embodiment, the L-asparaginase has at least 80% identity to the amino acid sequence of SEQ ID NO: 3, *e.g.* at least 81, 82, 83, 84, 85, 86, 87, 88, 89, 90, 91, 92, 93, 94, 95, 96, 97, 98, 99 or 100% identity to the amino acid sequence of SEQ ID NO: 3.

In one embodiment, the L-asparaginase is *Pseudomonas fluorescens* L-asparaginase. A reference *Pseudomonas fluorescens* L-asparaginase sequence is provided by SEQ ID NO: 4:

MQSANNVMVLYTGGTIGMQASANGLAPASGFEVRMREQFADADLPAWRFREMSP

LIDSANMNPAYWQRLRSAVVEAVDAGCDAVLILHGTDTLAYSAAAMSFQLLGLPAP

VVFTGSMLPAGVPDSDAWENVSGALTALAEGLEPGVHLYFHGALMAPTRCAKIRS

FGRNPFAALQRKDDFARAETLPAALDYRQPKALANVGVLPLIPGFDAAQLDAIISSGI

QGLVLECFGSGTGPSDNPQFLASLQRAQDQGVVVVAITQCHEGGVELDVYEAGSR

LRGAGVLSGAGMTREAAFGKLHALLGAGLAVEEVRRLVELDLHTNPT **(SEQ ID NO: 4)**

In one embodiment, the L-asparaginase has at least 80% identity to the amino acid sequence of SEQ ID NO: 4, *e.g.* at least 81, 82, 83, 84, 85, 86, 87, 88, 89, 90, 91, 92, 93, 94, 95, 96, 97, 98, 99 or 100% identity to the amino acid sequence of SEQ ID NO: 4.

In one embodiment, the L-asparaginase is *Wolinella succinogenes* L-asparaginase. A reference *Wolinella succinogenes* L-asparaginase sequence is provided by SEQ ID NO: 5:

MAKPQVTILATGGTIAGSGESSVKSSYSAGAVTVDKLLAAVPAINDLATIKGEQISSI
GSQEMTGKVWLKLAKRVNELLAQKETEAVIITHGTDTMEETAFFLNLTVKSQKPVVL
VGAMRSGSSMSADGPMNLYNAVNVAINKASTNKGVVIVMNDEIHAAREATKLNTTA
VNAFASPNTGKIGTVYYGKVEYFTQSVRPHTLASEFDISKIEELPRVDILYAHPDDTD
VLVNAALQAGAKGIIHAGMGNGNPFPLTQNALEKAAKSGVVVARSSRVGSGSTTQ
EAEVDDKKLGFVATESLNPQKARVLLMLALTKTSDREAIQKIFSTY **(SEQ ID NO: 5)**

In one embodiment, the L-asparaginase has at least 80% identity to the amino acid sequence of SEQ ID NO: 5, e.g. at least 81, 82, 83, 84, 85, 86, 87, 88, 89, 90, 91, 92, 93, 94, 95, 96, 97, 98, 99 or 100% identity to the amino acid sequence of SEQ ID NO: 5.

In one embodiment, the conjugate comprises a fragment of L-asparaginase, such as a fragment of SEQ ID NO: 2, 3, 4 or 5. In one embodiment, the recombinant heterologously-expressed L-asparaginase comprises a fragment of L-asparaginase, such as a fragment of SEQ ID NO: 2, 3, 4 or 5. In one embodiment, the fragment comprises at least 30 consecutive amino acids of the reference L-asparaginase amino acid sequence, e.g. at least 30, 40, 50, 60, 70, 80, 90, 100, 110, 120, 130, 140, 150, 160, 170, 180, 190, 200, 210, 220, 230, 240, 250, 260, 270, 280, 290, 300, 310 or more consecutive amino acids of the reference L-asparaginase amino acid sequence. In one embodiment, the reference L-asparaginase amino acid sequence is a native L-asparaginase amino acid sequence publically-available on an online database. According to the invention, fragments of L-asparaginase have an in vitro activity of at least 60% of the in vitro activity of the full-length L-asparaginase, e.g. at least 60%, 70%, 80%, 90%, 95% or 100% of the in vitro activity of the full-length L-asparaginase.

Conjugates of the invention comprise a water-soluble polymer.

Typically, the water-soluble polymer provides an expanded hydrodynamic volume, as compared to the un-conjugated L-asparaginase. PEGylation and PASylation are well-known to provide an expanded hydrodynamic volume. Hydrodynamic volumes may be assessed using any suitable method in the art, for example by analytical size exclusion chromatography and dynamic light scattering measurements. Without wishing to be bound by theory, the inventors believe that an increased hydrodynamic volume and correspondingly increased water shielding helps increase the in vivo $t_{1/2}$, whilst reducing immunogenicity of the conjugated L-asparaginase.

In one embodiment, the water-soluble polymer is selected from the group consisting of: (a) poly alkylene oxides; (b) poly amino acids; and (c) polysaccharides.

Typically, the water-soluble polymer is non-toxic.

In one embodiment, the water-soluble polymer comprises PEG. In one embodiment, the water-soluble polymer consists of PEG. PEG is very well-known in the art and is available in a variety of different configurations (typically linear or branched (including multi-arm)) and in a variety of different weights. PEG weights are typically within the range of 500 Da to 20000 Da. PEG mixtures are polydisperse, but current commercial PEG mixtures typically display low polydispersity, with polydispersity index (PDI) values approaching 1 (e.g. PDI = 1.05, 1.1, 1.15 or 1.2). When used in the context of PEG weights, the term "about" reflects this polydispersity.

In one embodiment, the PEG has a molecular weight of less than about 10000 Da, e.g. less than about 10000 Da, less than about 9000 Da, less than about 8000 Da, less than about 7000 Da, less than about 6000 Da, less than about 5000 Da, less than about 4000 Da, less than about 3000 Da, less than about 2000 Da, less than about 1000 Da, less than about 800 Da. In one embodiment, the PEG has a molecular weight of less than about 5000 Da.

In one embodiment, the PEG has a molecular weight of about 10000 Da. In one embodiment, the PEG has a molecular weight of about 5000 Da. In one embodiment, the PEG comprises linear 5000 Da PEG chains. In one embodiment, the PEG comprises branched 5000 Da PEG chains.

Methods of conjugating PEG to proteins are well-known in the art, and typically involve chemical conjugation. Typically, the PEG contains an activated/functionalised moiety that reacts preferentially with amino acids in the protein. The functional moiety is typically selected based upon the availability of reactive sites within the protein (such as lysine, cysteine, aspartic acid, glutamic acid and the N-terminus). In one embodiment, the PEG contains an active ester, such as succinimidyl ester. In one embodiment, the PEG contains a carbonate moiety, such as succinimidyl carbonate.

Numerous homobifunctionalized, heterobifunctionalized, and mono-methoxy endcapped monofunctionalized PEGs are commercially available. In one embodiment, the PEG is methoxyPEG (mPEG), such as functionalised mPEG. In one embodiment, the PEG is hydroxyPEG (HO-PEG), such as functionalised HO-PEG.

In one embodiment, PEG is covalently linked to one or more amino acids of L-asparaginase. In one embodiment, PEG is covalently linked to one or more amino acids of L-asparaginase by an amide bond.

In one embodiment, the conjugate of the invention has the formula:
PEG-O-CO-NH-[L-Asparaginase]

The conjugate of the invention may comprise multiple PEG moieties, as represented by the following formula: $(PEG-O-CO-NH)_n$-[L-Asparaginase] wherein n = 1-30, typically 5-15.

In one embodiment, the water-soluble polymer comprises a PAS polymer. In one embodiment, the water-soluble polymer consists of a PAS polymer. PAS polymers are conformationally-disordered polypeptide chains comprising Proline (P), Alanine (A) and Serine (S). PAS polymers display biophysical properties that are similar to PEG, and conjugation to PAS polymers ("PASylation") also extends *in vivo* $t_{1/2}$ and reduces the immunogenicity of conjugated proteins.

An advantage of PASylation over PEGylation is that PAS polymers may be fused to L-asparaginase during recombinant expression. Put another way, L-asparaginase and the PAS polymer may be expressed as a single polypeptide. Advantageously, PASylation avoids the requirement for a separate conjugation step (as required by PEGylation) thereby simplifying production of the conjugates of the invention. Thus, in one embodiment, conjugate of the invention comprises L-asparaginase and a PAS polymer expressed as a single polypeptide chain.

The skilled person will be aware that proline is encoded by codons: "CCU", "CCC", "CCA" and "CCG"; that alanine is encoded by codons: "GCU", "GCC", "GCA" and "GCG"; and that serine is encoded by codons: "UCU", "UCC", "UCA", "UCG", "AGU" and "AGC".

In one embodiment, at least 80% of the amino acid residues in the PAS polymer consist of proline, alanine and serine, e.g. at least 80%, 85%, 90%, 95%, 97%, 99% or 100% of the amino acid residues in the PAS polymer are selected from the group consisting of proline, alanine and serine.

In one embodiment, the PAS polymer comprises at least 10 amino acid residues, e.g. at least 15, 20, 25 or 30 amino acid residues.

In one embodiment, the PAS polymer comprises 10-60 amino acid residues. In one embodiment, the PAS polymer comprises 15-50 amino acid residues. In one embodiment, the PAS polymer comprises 20-40 amino acid residues. In one embodiment, the PAS polymer comprises 20-30 amino acid residues.

In one embodiment, at least 80% of the amino acid residues in the PAS polymer consist of proline, alanine and serine, and the PAS polymer comprises 10-60 amino acid residues.

In one embodiment, the PAS polymer additionally comprises a purification tag. In one embodiment, the purification tag is a Hiss-tag. In one embodiment, the purification tag is a Strep-tag.

In one embodiment, the PAS polymer is positioned at the N-terminus of the L-asparaginase. In one embodiment, the PAS polymer is positioned at the C-terminus of the L-asparaginase. In one embodiment, the PAS polymers are positioned at the N- and C-termini of the L-asparaginase.

In one embodiment, the PAS polymer and the L-asparaginase are linked via a linker. In one embodiment, the spacer is an amino acid linker. Typically, a linker comprises up to about 20-25 amino acid residues. Thus, in one embodiment, conjugate of the invention comprises L-asparaginase, PAS polymer and one or more linkers expressed as a single polypeptide chain.

L-asparaginases of the invention are expressed in a host cell other than *E. coli.*

Suitable host cells of the bacterial genera include, but are not limited to, cells of *Erwinia, Pseudomonas, Bacillus, Lactobacillus,* and *Streptomyces.* Suitable cells of bacterial species include, but are not limited to, cells of *Erwinia chrysanthemi, Pseudomonas fluorescens, Pseudomonas putida, Pseudomonas aeruginosa, Bacillus subtilis, Bacillus licheniformis, Lactobacillus brevis* and *Streptomyces lividans.* In one embodiment, the host cell is selected from the list consisting of *Pseudomonas aeruginosa, Pseudomonas fluorescens* and *Pseudomonas putida.* In one embodiment, the host cell is *Erwinia chrysanthemi.*

Suitable host cells of filamentous fungi include all filamentous forms of the subdivision Eumycotina. Suitable cells of filamentous fungal genera include, but are not limited to, cells of *Acremonium, Aspergillus, Aureobasidium, Bjerkandera, Ceriporiopsis, Chrysoporium, Coprinus, Coriolus, Corynascus, Chaetomium, Cryptococcus, Filobasidium, Fusarium, Gibberella, Humicola, Hypocrea, Magnaporthe, Mucor, Myceliophthora, Neocallimastix, Neurospora, Paecilomyces, Penicillium, Phanerochaete, Phlebia, Piromyces, Pleurotus, Scytaldium, Schizophyllum, Sporotrichum, Talaromyces, Thermoascus, Thielavia, Tolypocladium, Trametes,* and *Trichoderma.* In certain aspects, the recombinant cell is a *Trichoderma sp.* (*e.g., Trichoderma reesei*), *Penicillium sp., Humicola sp.* (*e.g., Humicola insolens*); *Aspergillus sp.* (*e.g., Aspergillus niger*), *Chrysosporium sp., Fusarium sp.,* or *Hypocrea sp.* Suitable cells can also include cells of various anamorph and teleomorph forms of these filamentous fungal genera.

Suitable cells of filamentous fungal species include, but are not limited to, cells of *Aspergillus awamori, Aspergillus fumigatus, Aspergillus foetidus, Aspergillus japonicus, Aspergillus nidulans, Aspergillus niger, Aspergillus oryzae, Chrysosporium lucknowense, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Bjerkandera adusta,*

*Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Coprinus cinereus, Coriolus hirsutus, Humicola insolens, Humicola lanuginosa, Mucor miehei, Myceliophthora thermophila, Neurospora crassa, Neurospora intermedia, Penicillium purpurogenum, Penicillium canescens, Penicillium solitum, Penicillium funiculosum, Phanerochaete chrysosporium, Phlebia radiate, Pleurotus eryngii, Talaromyces flavus, Thielavia terrestris, Trametes villosa, Trametes versicolor, Trichoderma harzianum, Trichoderma koningii, Trichoderma longibrachiatum, Trichoderma reesei,* and *Trichoderma viride.*

The host cell is typically a prokaryotic host cell.

In one embodiment, the L-asparaginase is recombinantly expressed. Nucleic acid encoding the L-asparaginase is typically operably linked to one or more nucleic acid sequences capable of providing for or aiding the transcription and/or translation of the L-asparaginase, for example a promoter operable in the organism in which the L-asparaginase is to be expressed. The promoters can be homologous or heterologous, and constitutive or inducible. Promoter sequences are well-known in the art.

Where recombinant expression in a filamentous fungal host is desired, the promoter can be a fungal promoter (including but not limited to a filamentous fungal promoter), a promoter operable in plant cells, a promoter operable in mammalian cells.

In one embodiment, the L-asparaginase is endogenously expressed by the host cell.

In one embodiment, the L-asparaginase is produced synthetically (typically without involving use of a host cell). Synthetic production of L-asparaginase substantially avoids the presence of HCPs in compositions of the invention.

L-asparaginase can be recovered and/or purified from the host cell by any method known in the art, for example, by chromatography (*e.g.,* ion (anion/cation) exchange, affinity - including nickel affinity and glutathione affinity, and sizing column chromatography), centrifugation, differential solubility, or by any other standard technique for the purification of proteins. In addition, the L-asparaginase can be fused to heterologous polypeptide sequences to facilitate purification. Such purification tags are known in the art and any conventional tag may be used.

A patient that has previously been administered *E. coli*-derived L-asparaginase may be readily identified *e.g.* by reviewing the patient's medical record, which will typically indicate that the patient has previously been administered Oncaspar®.

In one embodiment, the patient is undergoing treatment with *E. coli*-derived L-asparaginase but is not identified as having a hypersensitivity to *E. coli*-derived L-asparaginase.

In one embodiment, the patient is identified as having a hypersensitivity to an *E. coli*-derived L-asparaginase. Various types of hypersensitivity are known to the skilled person, and include *e.g.* allergic reaction, anaphylactic shock, and sub-clinical hypersensitivity (also known as silent inactivation).

A patient with a hypersensitivity to an *E. coli*-derived L-asparaginase may be identified by an allergic reaction following exposure to *E. coli*-derived L-asparaginase. Symptoms associated with an allergic reaction are well-known, and include, but are not limited to, skin irritation (*e.g.* transient flushing, rash, urticaria), difficulty breathing (*e.g.* bronchospasm, wheezing), vomiting, diarrhoea, oedema (*e.g.* angioedema) and hypotension.

A patient may have experienced anaphylactic shock in response to an *E. coli*-derived L-asparaginase. Anaphylactic shock is an allergic reaction that occurs rapidly following exposure and typically involves one or more symptoms including, but not limited to, throat and/or tongue swelling, vomiting, severe skin irritation and hypotension.

A patient may have sub-clinical hypersensitivity (also known as silent inactivation) to an *E. coli*-derived L-asparaginase. Sub-clinical hypersensitivity is characterised by an anti-*E.coli* L-asparaginase antibody response in the absence of clinical signs of hypersensitivity (such as those associated with allergic reactions, as described above). A patient with sub-clinical hypersensitivity may be identified as a patient who developed anti-*E. coli* L-asparaginase antibodies. Development of anti-*E. coli*-derived L-asparaginase antibodies can be identified by various methods known in the art, *e.g.* enzyme-linked immunosorbent assays (ELISA).

Sub-clinical hypersensitivity may also be identified as a progressive worsening of patient clinical symptoms (*e.g.* of ALL) despite treatment with *E. coli*-derived L-asparaginase. Sub-clinical hypersensitivity may also be identified by assessing serum L-asparaginase activity over time.

The patient is typically a mammal, preferably a human.

In one embodiment, the patient is 21 years old or younger. In one embodiment, the patient is 18 years old or younger. In one embodiment, the patient is 15 years old or younger. In one embodiment, the patient is 12 years old or younger. In one embodiment, the patient is 8 years old or younger. In one embodiment, the patient is 6 years old or younger. In one embodiment, the patient is 4 years old or younger. In one embodiment, the patient is 2 years old or younger. In one embodiment, the patient is 22 years old or older.

Methods of determining L-asparagine aminohydrolase activity are well-known in the art.

In one embodiment, the conjugate of the invention has an *in vitro* activity of at least 80% as compared to an equivalent conjugate that was expressed in *E. coli, e.g.* at least 80%, 90% or 100% of the *in vitro* activity of an equivalent conjugate that was expressed in *E. coli.*

In one embodiment, L-asparagine aminohydrolase activity is measured using the Nesslerisation method, which determines the amount of ammonia that is liberated by the catalytic activity of L-asparaginase upon L-asparagine. For example, test samples may be prepared as 900µL of Tris-HCL buffer and L-asparagine at pH 8.6. L-asparaginase (100µL) may be added to the test sample and incubated for 30 minutes at 37°C. After 30 minutes, the reaction may be quenched by adding 1.5M trichloroacetic acid solution. Samples are then centrifuged to remove any particulates. 100µL of supernatant may then be added to tubes containing 3.8mL of water and Nessler's reagent, and incubated for 15 minutes. Samples are then analysed spectrophotometrically (at 425nm) to provide an indication of the relative amount of ammonia liberated by catalysis of L-asparagine by L-asparaginase. The Nesslerisation method is ideally-suited to the assessment of *in vitro* L-asparaginase activity.

Another method for determining L-asparagine aminohydrolase activity involves incubating L-asparaginase with L-aspartic β-hydroxamate. L-aspartic β-hydroxamate is catalysed to yield L-asparagine and hydroxylamine, which is then condensed with 8-hydroxyquinoline and oxidised to indooxine. L-asparagine aminohydrolase activity is determined spectrophotometrically (at 710 nm) (see *e.g.* Lanvers et al. Analytical Biochemistry (2002), 309(1):117-126). The L-aspartic β-hydroxamate catalysis method is ideally-suited to the assessment of L-asparaginase activity in bodily fluid samples, such as serum. The L-aspartic β-hydroxamate catalysis method is ideally-suited to the assessment of *in vivo* $t_{1/2}$ *e.g.* by assessing L-asparaginase activity in plasma samples taken at intervals following administration of L-asparaginase (including administration of conjugate of the invention).

Conjugates of the invention typically have a longer *in vivo* $t_{1/2}$ than when the L-asparaginase is not conjugated to a water-soluble polymer, when administered at an equivalent protein dose (*i.e.* weight of protein administered: bodyweight). In one embodiment, the conjugate comprises L-asparaginase from *E. chrysanthemi* and has a longer $t_{1/2}$ than EwASNase. In one embodiment, wherein the L-asparaginase is from *E. chrysanthemi* and the water-soluble polymer comprises PEG, the conjugate has a longer $t_{1/2}$ than EwASNase. In one embodiment, wherein the L-asparaginase is from *E. chrysanthemi* and the water-soluble polymer comprises a PAS polymer, the conjugate has a longer $t_{1/2}$ than EwASNase.

In one embodiment, the conjugate comprises L-asparaginase from *E. chrysanthemi* and has a longer $t_{1/2}$ than EcASNase, when administered at an equivalent protein dose (*i.e.* weight of protein administered: bodyweight). In one embodiment, wherein the L-asparaginase is from *E. chrysanthemi* and the water-soluble polymer comprises PEG, the conjugate has a longer $t_{1/2}$ than EcASNase. In one embodiment, wherein the L-asparaginase is from *E. chrysanthemi* and the water-soluble polymer comprises a PAS polymer, the conjugate has a longer $t_{1/2}$ than EcASNase.

In one embodiment, the conjugate comprises L-asparaginase from *E. chrysanthemi* and has a similar or longer $t_{1/2}$ than Oncaspar®, when administered at an equivalent protein dose (*i.e.* weight of protein administered: bodyweight). In one embodiment, wherein the L-asparaginase is from *E. chrysanthemi* and the water-soluble polymer comprises PEG, the conjugate has a similar or longer $t_{1/2}$ than Oncaspar®. In one embodiment, wherein the L-asparaginase is from *E. chrysanthemi* and the water-soluble polymer comprises a PAS polymer, the conjugate has a similar or longer $t_{1/2}$ than Oncaspar®. "Similar" $t_{1/2}$ refers to at least 70% of the $t_{1/2}$ of Oncaspar® *e.g.* at least 70%, 75%, 80%, 85%, 90%, 95%, 100%, 105%, 110%, 115%, 120%, 125% or at least 130% of the $t_{1/2}$ of Oncaspar®.

"Longer" $t_{1/2}$ refers to at least 10% longer *e.g.* at least 10, 20, 30, 40, 50, 60, 70, 80, 90, 100, 110, 120, 130, 140, 150, 160, 170, 180, 190, 200, 225, 250, 275 or at least 300% longer $t_{1/2}$

Conjugates and compositions of the invention are administered to a patient in a manner compatible with the dosage formulation, and in such amount as will be therapeutically effective (*i.e.* a "therapeutically effective amount").

Conjugates and compositions of the invention are typically administered as a second- or third- line therapy after treatment with *E. coli*-derived L-asparaginase.

Conjugates and compositions of the invention are generally administered by conventional routes *e.g.* intravenous, subcutaneous, intraperitoneal, or mucosal routes. The administration is typically by parenteral administration *e.g.* intravenous or intramuscular injection.

In one embodiment, conjugates (or recombinant heterologously-expressed L-asparaginases) of the invention are administered at a dose ranging from about 100 IU/m$^2$ to about 30000 IU/m$^2$ (about 0.2 - 60 mg protein/m$^2$). In one embodiment, conjugate of the invention is administered at a dose from about 100 IU/m$^2$ to about 2500 IU/m$^2$, such as about 100 IU/m$^2$ to about 500 IU/m$^2$, or about 500 IU/m$^2$ to about 2500 IU/m$^2$. In one embodiment, recombinant heterologously-expressed L-asparaginase is administered at a dose from about 6000 IU/m$^2$ to about 25000 IU/m$^2$, such as about 6000 IU/m$^2$ to about 10000 IU/m$^2$, or about 10000 IU/m$^2$ to about 25000 IU/m$^2$. In pediatric patients aged ≤21 years, conjugates of the invention are typically administered at a lower dose than in patients aged >21 years.

In one embodiment, treatment comprises administration of conjugates or compositions of the invention less than three times per week. In one embodiment, treatment comprises administration of conjugates or compositions of the invention less than two times per week. In one embodiment, treatment comprises administration of conjugates or compositions of the invention less than one time per week. In one embodiment, treatment comprises administration of conjugates or compositions of the invention at at least 7-day intervals, *e.g.* at least 7, 8, 9, 10, 11, 12, 13, 14, 15, 16, 17, 18, 19, 20 or 21 day intervals.

In one embodiment, serum L-asparaginase activity is measured prior to the next administration of conjugate or composition of the invention. In one embodiment, the administered dose is increased to raise serum L-asparaginase activity to a desired level. In one embodiment, the administration interval is decreased to raise serum L-asparaginase activity to a desired level. In one embodiment, the administered dose is increased and the administration interval is decreased to raise serum L-asparaginase activity to a desired level. The desired serum L-asparaginase activity level is typically a level which provides a therapeutic reduction of asparagine.

In one embodiment, conjugates or compositions of the invention are administered as a monotherapy. In one embodiment, conjugates or compositions of the invention are administered as part of a combination therapy *e.g.* in combination with other chemotherapy or radiotherapy.

In one embodiment, the disease treatable by L-asparagine depletion is a cancer. In one embodiment, the cancer is selected from the group consisting of Acute Lymphoblastic Leukemia (ALL), lymphosarcoma, non-Hodgkin's lymphoma, NK lymphoma, and pancreatic cancer. In one embodiment, the cancer is ALL.

There are many established algorithms available to align two amino acid sequences. Typically, one sequence acts as a reference sequence, to which test sequences may be compared. The sequence comparison algorithm calculates the percentage sequence identity for the test sequence(s) relative to the reference sequence, based on the designated program parameters. Alignment of amino acid sequences for comparison may be conducted, for example, by computer implemented algorithms (*e.g.* GAP, BESTFIT, FASTA or TFASTA), or BLAST and BLAST 2.0 algorithms.

The BLOSUM62 table shown below is an amino acid substitution matrix derived from about 2,000 local multiple alignments of protein sequence segments, representing highly conserved regions of more than 500 groups of related proteins (Henikoff & Henikoff, Proc. Natl. Acad. Sci. USA 89:10915-10919, 1992; incorporated herein by reference). Amino acids are indicated by the standard one-letter codes. The percent identity is calculated as:

$$\frac{\text{Total number of identical matches}}{[\text{length of the longer sequence plus the number of gaps introduced into the longer sequence in order to align the two sequences}]} \times 100$$

BLOSUM62 table

|   | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 |   |   |   |   |   |   |   |   |   |   |   |   |   |   |   |   |   |   |   |
| R | -1 | 5 |   |   |   |   |   |   |   |   |   |   |   |   |   |   |   |   |   |   |
| N | -2 | 0 | 6 |   |   |   |   |   |   |   |   |   |   |   |   |   |   |   |   |   |
| D | -2 | -2 | 1 | 6 |   |   |   |   |   |   |   |   |   |   |   |   |   |   |   |   |
| C | 0 | -3 | -3 | -3 | 9 |   |   |   |   |   |   |   |   |   |   |   |   |   |   |   |
| Q | -1 | 1 | 0 | 0 | -3 | 5 |   |   |   |   |   |   |   |   |   |   |   |   |   |   |
| E | -1 | 0 | 0 | 2 | -4 | 2 | 5 |   |   |   |   |   |   |   |   |   |   |   |   |   |
| G | 0 | -2 | 0 | -1 | -3 | -2 | -2 | 6 |   |   |   |   |   |   |   |   |   |   |   |   |
| H | -2 | 0 | 1 | -1 | -3 | 0 | 0 | -2 | 8 |   |   |   |   |   |   |   |   |   |   |   |
| I | -1 | -3 | -3 | -3 | -1 | -3 | -3 | -4 | -3 | 4 |   |   |   |   |   |   |   |   |   |   |
| L | -1 | -2 | -3 | -4 | -1 | -2 | -3 | -4 | -3 | 2 | 4 |   |   |   |   |   |   |   |   |   |
| K | -1 | 2 | 0 | -1 | -3 | 1 | 1 | -2 | -1 | -3 | -2 | 5 |   |   |   |   |   |   |   |   |
| M | -1 | -1 | -2 | -3 | -1 | 0 | -2 | -3 | -2 | 1 | 2 | -1 | 5 |   |   |   |   |   |   |   |
| F | -2 | -3 | -3 | -3 | -2 | -3 | -3 | -3 | -1 | 0 | 0 | -3 | 0 | 6 |   |   |   |   |   |   |
| P | -1 | -2 | -2 | -1 | -3 | -1 | -1 | -2 | -2 | -3 | -3 | -1 | -2 | -4 | 7 |   |   |   |   |   |
| S | 1 | -1 | 1 | 0 | -1 | 0 | 0 | 0 | -1 | -2 | -2 | 0 | -1 | -2 | -1 | 4 |   |   |   |   |
| T | 0 | -1 | 0 | -1 | -1 | -1 | -1 | -2 | -2 | -1 | -1 | -1 | -1 | -2 | -1 | 1 | 5 |   |   |   |
| W | -3 | -3 | -4 | -4 | -2 | -2 | -3 | -2 | -2 | -3 | -2 | -3 | -1 | 1 | -4 | -3 | -2 | 11 |   |   |
| Y | -2 | -2 | -2 | -3 | -2 | -1 | -2 | -3 | 2 | -1 | -1 | -2 | -1 | 3 | -3 | -2 | -2 | 2 | 7 |   |
| V | 0 | -3 | -3 | -3 | -1 | -2 | -2 | -3 | -3 | 3 | 1 | -2 | 1 | -1 | -2 | -2 | 0 | -3 | -1 | 4 |

In a homology comparison, the identity may exist over a region of the sequences that is at least 10 amino acid residues in length (e.g. at least 15, 20, 25, 30, 35, 40, 45, 50, 60, 70, 80, 90, 100, 125, 150, 175, 200, 225, 250, 275, 300, 325 or more amino acid residues in length - *e.g.* up to the entire length of the reference sequence.

Substantially homologous polypeptides have one or more amino acid substitutions, deletions, or additions. In many embodiments, those changes are of a minor nature, for example, involving only conservative amino acid substitutions. Conservative substitutions are those made by replacing one amino acid with another amino acid within the following groups: Basic: arginine, lysine, histidine; Acidic: glutamic acid, aspartic acid; Polar: glutamine, asparagine; Hydrophobic: leucine, isoleucine, valine; Aromatic: phenylalanine, tryptophan, tyrosine; Small: glycine, alanine, serine, threonine, methionine. Substantially homologous polypeptides also encompass those comprising other substitutions that do not significantly affect the folding or activity of the polypeptide; small deletions, typically of 1 to about 30 amino acids (such as 1-10, or 1-5 amino acids); and small amino- or carboxyl-terminal extensions, such as an amino-terminal methionine residue, a small linker peptide of up to about 20-25 residues, or an affinity tag.

In one embodiment, the composition of the invention is substantially free from host cell proteins from *E. coli.* In one embodiment, the composition of the invention does not contain host cell proteins from *E. coli.*

Due to its high detection sensitivity, Enzyme Linked Immunosorbent Assay (ELISA) is the current gold standard method for detecting HCPs. HCP detection protocols are well-known in the art, and ELISA-based HCP detection assays are commercially available (*e.g.* "*E. coli* HCP ELISA Kit" from Cygnus Technologies, US (part of Marvai LifeSciences); and "*E. coli* HCP ELISA Kit (host cell protein)" from Abcam, U K).

There are, however, certain limitations associated with ELISA-based detection of HCPs. For example, anti-HCP antibody pools cannot cover the entire HCP population, and weakly immunogenic HCPs may not elicit any/enough antibodies to facilitate their detection. Through careful identification of suitable host cells and L-asparaginase sources, the present invention helps avoid the requirement for ultra-pure L-asparaginase preparations for the treatment of patients who have developed a hypersensitive reaction to Oncaspar® (or EcASNase) and/or patients who have developed an anti-L-asparaginase antibody response to Oncaspar® (or EcASNase), but do not display overt allergic symptoms.

Compositions of the invention may comprise excipients which are pharmaceutically acceptable and compatible with the active ingredient. Suitable excipients are, for example, water, saline, dextrose, glycerol, ethanol, or the like and combinations thereof. In addition, compositions of the invention may contain auxiliary substances such as wetting or emulsifying agents, and/or pH buffering agents.

In one embodiment, the conjugate or composition of the invention is in lyophilised form. In one embodiment, compositions of the invention are in lyophilised form. Conjugates or compositions of the invention may be lyophilised to provide a powdered form of the conjugate or composition. Lyophilised conjugates or compositions of the invention may then be reconstituted prior to administration. Sterile powders for the preparation of injectable solutions may be generated by lyophilising a solution comprising conjugates or compositions of the invention to yield a powder comprising the conjugate (or recombinant heterologously-expressed L-asparaginase) along with any optional co-solubilised biocompatible ingredients. Generally, dispersions or solutions are prepared by incorporating conjugate (or recombinant heterologously-expressed L-asparaginase) of the invention into a sterile vehicle that contains a basic dispersion medium or solvent (*e.g.,* a diluent) and, optionally, other biocompatible ingredients. A compatible diluent is one which is pharmaceutically acceptable (safe and non-toxic for administration to a human) and is useful for the preparation of a liquid formulation, such as a formulation reconstituted after lyophilisation. Diluents include *e.g.* sterile water, bacteriostatic water for injection, a pH buffered solution (*e.g.* phosphate-buffered saline), sterile saline solution, Ringer's solution or dextrose solution. Diluents may include *e.g.* aqueous solutions of salts and/or buffers.

In one embodiment, composition of the invention comprises one or more lyoprotectant(s). In one embodiment, conjugate or composition of the invention is lyophilised in combination with a lyoprotectant *e.g.* an amino acid such as monosodium glutamate or histidine; a methylamine such as betaine; a lyotropic salt such as magnesium sulfate; a polyol such as trihydric or higher molecular weight sugar alcohols, *e.g.* glycerin, dextran, erythritol, glycerol, arabitol, xylitol, sorbitol, and mannitol; propylene glycol; and combinations thereof. Additional exemplary lyoprotectants include glycerin and gelatin, and the sugars mellibiose, melezitose, raffinose, mannotriose and stachyose. In one embodiment, the lyoprotectant comprises trehalose. In one embodiment, the lyoprotectant comprises sucrose. In one embodiment, the lyoprotectant comprises trehalose and sucrose.

Lyoprotectants are added to the composition in a "protecting amount" (*e.g.* pre-lyophilisation) which means that the conjugate (or recombinant heterologously-expressed L-asparaginase) of the invention essentially retains its physical and chemical stability and integrity during storage (*e.g.,* after reconstitution and storage).

Conjugates and compositions of the invention may be prepared as injectables, either as liquid solutions or suspensions. Solid forms suitable for solution in, or suspension in, liquid prior to injection may alternatively be prepared.

In one embodiment, the composition is in dosage form.

In one embodiment, the composition is sterile.

Compositions of the invention may comprise buffering agents. In one embodiment, the buffering agent comprises histidine hydrochloride (*e.g.,* L-histidine HCL).

Compositions of the invention may comprise nonionic surfactant(s) such as polysorbate 20, polysorbate 40, polysorbate 60 or polysorbate 80 as stabilizing agents.

The invention also provides a method for producing a conjugate of the invention.

In one embodiment, the invention provides a method for producing a conjugate of the invention, the method comprising:

(a) expressing in a host cell other than *E. coli* nucleic acid encoding: (i) L-asparaginase from a source other than *E. coli* and (ii) a PAS polymer; and
(b) purifying the conjugate.

In one embodiment, the invention provides a method for producing a conjugate of the invention, the method comprising:

(a) expressing in a host cell other than *E. coli* nucleic acid encoding L-asparaginase from a source other than *E. coli;*
(b) purifying the L-asparaginase; and
(c) conjugating the L-asparaginase to PEG.

In one embodiment, the invention provides a method for producing a conjugate of the invention, the method comprising:

(a) purifying native L-asparaginase from a source other than *E. coli;* and
(b) conjugating the L-asparaginase to PEG.

The invention also provides a method for producing a recombinant heterologously-expressed L-asparaginase of the invention, the method comprising:

(a) expressing in a heterologous host cell other than *E. coli* nucleic acid encoding L-asparaginase from a source other than *E. coli;* and
(b) purifying the L-asparaginase.

## Brief description of the Figures

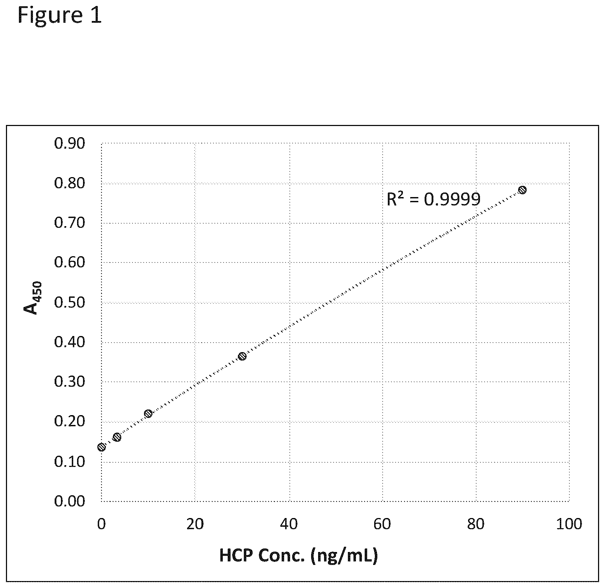

**Figure 1** Standard curve for the determination of *E. coli* HCPs in Oncaspar®. HCP concentration (ng/mL) is plotted on the X-axis against absorbance ($A_{450}$) on the Y-axis. $R^2$= 0.9999.

## Examples

### Example 1: Detection of *E. coli* HCPs in Oncaspar®

A commercial vial of Oncaspar® was analysed for the presence of *E. coli* HCPs by enzyme-linked immunosorbent assay (ELISA) and by mass spectrometry. Both analyses confirmed the presence of *E. coli* HCPs in Oncaspar®.

### (a) Detection of *E. coli* HCPs by ELISA

A commercial vial of Oncaspar® (3,750 IU) was reconstituted in water and analysed using anti-*E. coli* HCP ELISA. In the test kit, the ELISA plate was pre-coated with capture antibodies (anti-*E.coli* HCP antibodies). Following incubation of Oncaspar® with capture antibodies, detection antibodies (second anti- *E. coli* HCP antibodies, conjugated with biotin) were applied. Following incubation with detection antibodies, the plate was incubated with Streptavidin-Horseradish Peroxidase (HRP) conjugate, which binds to biotin-labelled antibody. "TMB" (3,3',5,5"-tetramethylbenzidine) substrate was then added, which is converted by the captured HRP to a coloured product in proportion to the amount of HCP bound to the plate. Absorbance was measured at 450nm.

A standard curve was generated to determine the relationship between the concentration of *E. coli* HCPs (ng/mL) and absorbance (405nm). The standard curve (Figure 1) had an $R^2$ value of 0.9999, which signifies a very good 'fit' and a high degree of confidence in the data.

ELISA analysis determined that the vial of Oncaspar® contains ~1.1 ng of *E. coli* HCPs.

A typical dose of Oncaspar® is 4500 IU, which corresponds to 1.2 vials of Oncaspar®. Accordingly, patients are administered ~1.3ng of *E. coli* HCPs every time they receive a dose of Oncaspar®.

(b) Detection of *E. coli* HCPs by mass spectrometry

Oncaspar® was also analysed using mass spectrometry (MS). Specifically, Liquid Chromatography with tandem mass spectrometry (LC-MS/MS) was used to identify unknown HCPs in Oncaspar® by Independent Data Acquisition ('IDA') (Patel, V.J. et al. Journal of Proteome Research, (2009). 8: 3752-3759). The sample was digested using trypsin, ionised and mass to charge ratios (m/z) of the ions were determined. Results were searched against the Uniprot complete protein database to identify HCPs in the sample.

Five main peptides were identified by MS, which are summarised in Table 1:

| Protein name | Species | Peptides (95%) |
|---|---|---|
| L-asparaginase | *E. coli* (strain K12) | 690 |
| L-asparaginase | *E. chrysanthemi* (strain 3937) | 43 |
| Putative sulfatase AslA | *E. coli* (strain K12) | 1 |
| Uncharacterised protein YggE_OS | *E. coli* (strain K12) | 1 |
| Protein UshA_OS | *E. coli* (strain K12) | 1 |

As expected, the most abundant peptide was identified as L-asparaginase from *E. coli.* The second most abundant peptide was identified as L-asparaginase from *E. chrysanthemi.* Without wishing to be bound by theory, the inventors believe that the peptides identified as L-asparaginase from *E. chrysanthemi* correspond to regions of *E. coli* L-asparaginase that have very high sequence identity to the native *E. chrysanthemi* L-asparaginase.

MS analysis also confirmed the presence of *E. coli* HCPs in Oncaspar®. These MS data further validate the results generated by ELISA. Of the *E. coli* HCPs in Oncaspar®, MS analysis identified (i) Putative sulfatase AslA; (ii) Uncharacterised protein YggE_OS; and (iii) Protein UshA_OS.

Example 2: Identification of highly immunogenic *E. coli* HCPs in Oncaspar®

The *E. coli* HCPs identified in Oncaspar® were assessed for their binding affinity to major histocompatibility complex class II (MHC II), and the likelihood of being presented by any known MHC II receptor. This is critical in the development of a T-cell response and provides a strong indicator of immunogenicity.

The immunogenicity analysis was performed using the publicly-available "NetMHCIIpan" server. This server uses Artificial Neural Networks and is trained on a dataset of over 500,000 measurements of binding affinity, and eluted ligand mass spectrometry, covering the three MHC II isotypes HLA-DR, HLA-DQ, HLA-DP, as well as mouse molecules (H-2) (for details see Jensen et al. 2018, Immunology, 154. 394-406).

The immunogenicity results are shown in Table 2:

| *E. coli* HCP | NetMHCIIan 3.2 | |
|---|---|---|
| | Number of strong-binders | No. of medium- plus strong- binders |
| Putative sulfatase AslA | 45 | 1322 |
| Uncharacterised protein YggE_OS | 124 | 1920 |
| Protein UshA_OS | 66 | 789 |

Strong binders have <50 nM affinity across 25 HLA alleles covering >99% of the human population. Medium-plus-strong binders have <500 nM affinity across 25 HLA alleles covering >99% of the human population (Wang *et al.,* 2010 BMC bioinformatics. 2010;11:568).

As shown in Table 2, the *E. coli* HCPs identified in Oncaspar® by MS displayed extremely high numbers of strong- and medium-plus-strong binders. Each of the *E. coli* HCPs identified in Oncaspar® is therefore highly immunogenic.

Administration of *E. coli* HCPs immunologically pre-disposes patients to elicit a hypersensitive response to subsequently administered L-asparaginase that is derived from *E. coli.* The risk of a dangerous and undesirable immune reaction is increased when the HCPs are highly immunogenic.

**Example 3: Production of a conjugate comprising *E. chrysanthemi* L-asparaginase and a PAS polymer**

Nucleic acid encoding *E. chrysanthemi* L-asparaginase and a PAS polymer is cloned into a pMMPc vector (GenBank accession number KC544266) under the control of a $P_c$ promoter. Cloning is confirmed by polymerase chain reaction analysis. *Pseudomonas fluorescens* strain MB214 is then transformed with the vector by electroporation.

Transformed *Pseudomonas fluorescens* is inoculated into broth and grown for 48h, followed by centrifugation. A series of chromatography and concentration steps are performed. Sample purity is confirmed by sodium dodecyl sulfate - polyacrylamide gel electrophoresis. The amino acid sequence of the conjugate is confirmed by N-terminal protein sequencing and liquid chromatography-mass spectrometry.

L-asparaginase activity is confirmed *in vitro* by the Nesslerisation method at 37°C.

**Example 4: Production of a conjugate comprising *E. chrysanthemi* L-asparaginase and 5000 Da PEG**

*Step A:* Nucleic acid encoding *E. chrysanthemi* L-asparaginase is cloned into a pMMPc vector as described in Example 3. *Pseudomonas fluorescens* strain BM214 is then transformed with the vector and grown as per Example 3. Following inoculation and growth for 48h, *E. chrysanthemi* L-asparaginase is then purified and concentrated as set out in Example 3.

*Step 8:* Purified *E. chrysanthemi* L-asparaginase (5 mg/ml) is then mixed in the presence of 5000 Da functionalised mPEG (100 mg/ml) and sodium phosphate buffer (100mM; pH 8.0) for 2.5 hours. The PEGylated *E. chrysanthemi* L-asparaginase is concentrated and purified, and L-asparaginase activity is confirmed *in vitro* by the Nesslerisation method at 37°C.

**Example 5: *In vivo* half-life analysis of *E. chrysanthemi* L-asparaginase conjugates**

To assess the pharmacokinetic properties of the conjugates of Examples 3 and 4, the conjugates are administered intravenously to immune competent mice ("Group 1" and "Group 2", respectively). As controls, "Group 3" mice are administered *E. chrysanthemi* L-asparaginase concentrated and purified in Example 3 Step A (*i.e.* not conjugated to PEG).

Blood is collected from the mice by retro-orbital bleeding. Bleedings are performed at 1hr pre-administration, and at 6h, 12h, 18h, 24h, 36h and 48h post-administration. Residual L-asparaginase activity is assessed by the L-aspartic β-hydroxamate catalysis method, and *in vivo* $t_{1/2}$ values are calculated.

Groups 1 and 2 display a significantly longer $t_{1/2}$ than Group 3.

Example 6: Suitability for use in patients who have developed hypersensitivity to Oncaspar®.

Patient #1: A male patient suffering from ALL experiences a hypersensitive reaction to his third dose of Oncaspar®. One week after this allergic reaction, he is intravenously administered conjugate prepared according to Example 3, at a dose of 750 IU/m². The patient tolerates the conjugate of *E. chrysanthemi* L-asparaginase and PAS polymer, and his SAA levels are within therapeutic range (≥0.1 IU/ml) at 48h after dosing.

Patient #2: A female patient suffering from ALL experiences an allergic reaction to her second dose of Oncaspar®. One week after this allergic reaction, she is intravenously administered conjugate prepared according to Example 3, at a dose of 750 IU/m². The patient tolerates the conjugate of *E. chrysanthemi* L-asparaginase and PAS polymer, and her SAA levels are within therapeutic range at 48h after dosing.

**CLAUSES**

1. A conjugate comprising L-asparaginase and a water-soluble polymer, for use in treating a disease treatable by L-asparagine depletion in a patient, wherein:

(a) the L-asparaginase is from a source other than *E. coli;*

(b) the L-asparaginase is expressed in a host cell other than *E. coli;* and

(c) the patient has previously been administered *E.* coli-derived L-asparaginase.

2. The conjugate for use according to clause 1, wherein the L-asparaginase is a recombinant L-asparaginase.

3. A recombinant heterologously-expressed L-asparaginase for use in treating a disease treatable by L-asparagine depletion in a patient, wherein:

(a) the L-asparaginase is from a source other than *E. coli;*

(b) the heterologous host cell is a host cell other than *E. coli;* and

(c) the patient has previously been administered *E. coli*-derived L-asparaginase.

4. The conjugate for use according to clause 1 or clause 2, or the recombinant heterologously-expressed L-asparaginase for use according to clause 3, wherein the patient has had a hypersensitivity to an *E. coli*-derived L-asparaginase.

5. The conjugate for use according to clause 4, or the recombinant heterologously-expressed L-asparaginase for use according to clause 4, wherein the hypersensitivity is selected from the group consisting of allergic reaction, anaphylactic shock, and silent inactivation.

6. The conjugate for use according to any one of clauses 1 to 5, or the recombinant heterologously-expressed L-asparaginase for use according to any one of clauses 1 to 5, wherein the *E. coli*-derived L-asparaginase is Oncaspar®.

7. The conjugate for use according to any one of clauses 1 to 6, or the recombinant heterologously-expressed L-asparaginase for use according to any one of clauses 1 to 6, wherein the L-asparaginase is *Erwinia* L-asparaginase.

8. The conjugate for use according to clause 7, or the recombinant heterologously-expressed L-asparaginase for use according to clause 7, wherein the *Erwinia* L-asparaginase is *E. chrysanthemi* L-asparaginase.

9. The conjugate for use according to clause 8, or the recombinant heterologously-expressed L-asparaginase for use according to clause 8, wherein the *E. chrysanthemi* L asparaginase has at least 80% identity to the amino acid sequence of SEQ ID NO: 2.

10. The conjugate for use according to any one of the preceding clauses, wherein the water-soluble polymer comprises polyethylene glycol (PEG).

11. The conjugate for use according to clause 10, wherein the PEG has a molecular weight of less than about 10000 Da.

12. The conjugate for use according to clause 10 or clause 11, wherein the PEG has a molecular weight of less than about 5000 Da

13. The conjugate for use according to any one of clauses 10 to 12, wherein the PEG has a molecular weight of less than about 4000 Da

14. The conjugate for use according to any one of clauses 10 to 13, wherein the PEG has a molecular weight of less than about 3000 Da

15. The conjugate for use according to any one of clauses 10 to 14, wherein the PEG has a molecular weight of less than about 2000 Da.

16. The conjugate for use according to any one of the preceding clauses, wherein the water-soluble polymer is a PAS polymer.

17. The conjugate for use according to clause 16, wherein at least 80% of the amino acid residues in the PAS polymer consist of proline, alanine and serine.

18. The conjugate for use according to clause 16 or clause 17, wherein at least 90% of the amino acid residues in the PAS polymer consist of proline, alanine and serine.

19. The conjugate for use according to any one of clauses 16 to 18, wherein at least 95% of the amino acid residues in the PAS polymer consist of proline, alanine and serine.

20. The conjugate for use according to any one of clauses 16 to 19, wherein at least 97% of the amino acid residues in the PAS polymer consist of proline, alanine and serine.

21. The conjugate for use according to any one of clauses 16 to 20, wherein at least 99% of the amino acid residues in the PAS polymer consist of proline, alanine and serine.

22. The conjugate for use according to any one of clauses 16 to 21, wherein all of the amino acid residues in the PAS polymer consist of proline, alanine and serine.

23. The conjugate for use according to any one of clauses 16 to 22, wherein the PAS polymer comprises at least 10 amino acid residues.

24. The conjugate for use according to any one of clauses 16 to 23, wherein the PAS polymer comprises at least 15 amino acid residues.

25. The conjugate for use according to any one of clauses 16 to 24, wherein the PAS polymer comprises at least 20 amino acid residues.

26. The conjugate for use according to any one of clauses 16 to 25, wherein the PAS polymer comprises at least 25 amino acid residues.

27. The conjugate for use according to any one of clauses 16 to 26, wherein the PAS polymer comprises at least 30 amino acid residues.

28. The conjugate for use according to any one of clauses 16 to 27, wherein the PAS polymer comprises 10-60 amino acid residues.

29. The conjugate for use according to any one of clauses 16 to 28, wherein the PAS polymer comprises 15-50 amino acid residues.

30. The conjugate for use according to any one of clauses 16 to 29, wherein the PAS polymer comprises 20-40 amino acid residues.

31. The conjugate for use according to any one of clauses 16 to 30, wherein the PAS polymer comprises 20-30 amino acid residues.

32. The conjugate for use according to any one of clauses 16 to 31, wherein the PAS polymer additionally comprises a purification tag.

33. The conjugate for use according to clause 32, wherein the purification tag is a $His_6$-tag.

34. The conjugate for use according to clause 32, wherein the purification tag is a Strep-tag.

35. The conjugate for use according to any one of the preceding clauses, or the recombinant heterologously-expressed L-asparaginase for use according to any one of the preceding clauses, wherein the host cell is of the genus *Pseudomonas.*

36. The conjugate for use according to clause 35, or the recombinant heterologously-expressed L-asparaginase for use according to clause 35, wherein the host cell is *Pseudomonas aeruginosa.*

37. The conjugate for use according to clause 35, or the recombinant heterologously-expressed L-asparaginase for use according to clause 35, wherein the host cell is *Pseudomonas fluorescens.*

38. The conjugate for use according to clause 35, or the recombinant heterologously-expressed L-asparaginase for use according to clause 35, wherein the host cell is *Pseudomonas putida.*

39. The conjugate for use according to any one of the preceding clauses, or the recombinant heterologously-expressed L-asparaginase for use according to any one of the preceding clauses, wherein the patient is 21 years old or younger.

40. The conjugate for use according to any one of the preceding clauses, or the recombinant heterologously-expressed L-asparaginase for use according to any one of the preceding clauses, wherein the conjugate, or the recombinant heterologously-expressed L-asparaginase is in lyophilised form.

41. The conjugate for use according to any one of the preceding clauses, wherein the conjugate has an *in vitro* activity of at least 80% as compared to an equivalent conjugate that was expressed in *E. coli.*

42. The conjugate for use according to any one of the preceding clauses, wherein the conjugate has an *in vitro* activity of at least 90% as compared to an equivalent conjugate that was expressed in *E. coli.*

43. The conjugate for use according to any one of the preceding clauses, wherein the conjugate has an *in vitro* activity that is at least as high as an equivalent conjugate that was expressed in *E. coli.*

44. The conjugate for use according to any one of the preceding clauses, or the recombinant heterologously-expressed L-asparaginase for use according to any one of the preceding clauses, wherein said treatment comprises intravenous administration of the conjugate.

45. The conjugate for use according to any one of the preceding clauses, or the recombinant heterologously-expressed L-asparaginase for use according to any one of the preceding clauses, wherein said treatment comprises intramuscular administration of the conjugate.

46. The conjugate for use according to any one of the preceding clauses, wherein said treatment comprises administration of the conjugate less than three times per week

47. The conjugate for use according to any one of clauses 1 to 46, wherein said treatment comprises administration of the conjugate less than two times per week.

48. The conjugate for use according to any one of clauses 1 to 47, wherein said treatment comprises administration of the conjugate less than one time per week.

49. The conjugate for use according to any one of the preceding clauses, or the recombinant heterologously-expressed L-asparaginase for use according to any one of the preceding clauses, wherein said disease treatable by L-asparagine depletion is a cancer.

50. The conjugate for use according to clause 49, or the recombinant heterologously-expressed L-asparaginase for use according to clause 49, wherein said cancer is selected from the group consisting of Acute Lymphoblastic Leukemia (ALL), non-Hodgkin's lymphoma, NK lymphoma, and pancreatic cancer.

51. The conjugate for use according to clause 49 or clause 50 , or the recombinant heterologously-expressed L-asparaginase according to clause 49 or clause 50, wherein said cancer is ALL.

52. A composition comprising: (a) the conjugate for use according to any one of the preceding clauses, and/or (b) the recombinant heterologously-expressed L-asparaginase for use according to any one of the preceding clauses, and a pharmaceutically acceptable excipient.

53. A composition for use according to clause 52, wherein the composition comprises one or more lyoprotectant(s).

54. The composition for use according to clause 53, wherein the lyoprotectant(s) are selected from the list consisting of trehalose and sucrose.

55. The composition for use according to any one of clauses 52 to 54, wherein the composition is in lyophilised form.

56. The composition for use according to any one of clauses 52 to 54, wherein the composition is in solubilised form.

57. A method of treating a disease treatable by L-asparagine depletion in a patient that has been previously administered E. coli-derived L-asparaginase, said method comprising administering to said patient an effective amount of the conjugate or composition as defined in any one of the preceding clauses.

58. A method of treating a disease treatable by L-asparagine depletion in a patient that has been previously administered *E. coli*-derived L-asparaginase, said method comprising administering to said patient an effective amount of the recombinant heterologously-expressed L-asparaginase or composition as defined in any one of the preceding clauses.

59. The method according to clause 57 or clause 58, wherein the patient has had a hypersensitivity to an *E. coli*-derived L-asparaginase.

60. The method according to clause 59, wherein the hypersensitivity is selected from the group consisting of allergic reaction, anaphylactic shock, and silent inactivation.

61. The method according to any one of clauses 57 to 60, wherein the patient has previously been administered Oncaspar®.

62. The method according to any one of clauses 57 to 60, wherein the patient is 21 years old or younger.

63. The method according to any one of clauses 57 to 62, wherein said disease treatable by L-asparagine depletion is a cancer.

64. The method according to clause 63, wherein said cancer is selected from the group consisting of Acute Lymphoblastic Leukemia (ALL), lymphosarcoma, non-Hodgkin's lymphoma, NK lymphoma, and pancreatic cancer.

65. The method according to clause 63 or clause 64, wherein said cancer is ALL.

66. The method according to any one of clauses 57 to 65, wherein said conjugate or composition is administered intravenously.

67. The method according to any one of clauses 57 to 66, wherein said conjugate or composition is administered intramuscularly.

68. The method according to any one of clauses 57 to 67, wherein said conjugate or composition is administered less than three times per week.

69. The method according to any one of clauses 57 to 68, wherein said conjugate or composition is administered less than two times per week.

70. The method according to any one of clauses 57 to 69, wherein said conjugate or composition is administered less than one time per week.

## Claims

1. A conjugate comprising L-asparaginase and a water-soluble polymer, for use in treating a disease treatable by L-asparagine depletion in a patient, wherein:

   (a) the L-asparaginase is from a source other than *E. coli;*
   (b) the L-asparaginase is expressed in a host cell other than *E. coli*; and
   (c) the patient has previously been administered *E.* coli-derived L-asparaginase;
   (d) the water-soluble polymer comprises polyethylene glycol (PEG);
   (e) the patient has been identified as having a hypersensitivity to *E. coli*-derived L-asparaginase; and
   (f) the L-asparaginase is *Erwinia* L-asparaginase;

   and wherein administration of the conjugate to the patient reduces the risk of further hypersensitive reactions, as compared to administration of *E. coli*-derived L-asparaginase.

2. The conjugate for use according to claim 1, wherein the L-asparaginase is a recombinant L-asparaginase.

3. The conjugate for use according to claim 1 or claim 2, wherein the hypersensitivity is selected from the group consisting of allergic reaction, anaphylactic shock, and silent inactivation.

4. The conjugate for use according to any one of claims 1 to 3, wherein the *E. coli*-derived L-asparaginase is Oncaspar®.

5. The conjugate for use according to any one of claims 1 to 4, wherein the *Erwinia* L-asparaginase is *E. chrysanthemi* L-asparaginase; optionally wherein the *E. chrysanthemi* L asparaginase has at least 80% identity to the amino acid sequence of SEQ ID NO: 2.

6. The conjugate for use according to any one of the preceding claims, wherein the PEG has a molecular weight of less than about 10000 Da; optionally less than about 5000 Da; optionally less than about 4000 Da; optionally less than about 3000 Da; optionally less than about 2000 Da.

7. The conjugate for use according to any one of the preceding claims, wherein the host cell is of the genus *Pseudomonas*; optionally wherein the host cell is:

   (a) *Pseudomonas aeruginosa*;
   (b) *Pseudomonas fluorescens*; or
   (c) *Pseudomonas putida.*

8. The conjugate for use according to any one of the preceding claims, wherein the patient is 21 years old or younger.

9. The conjugate for use according to any one of the preceding claims, wherein the conjugate is in lyophilised form.

10. The conjugate for use according to any one of the preceding claims, wherein the conjugate has an *in vitro* activity of at least 80% as compared to an equivalent conjugate that was expressed in *E. coli;* optionally wherein the conjugate has an *in vitro* activity of at least 90% as compared to an equivalent conjugate that was expressed in *E. coli;* optionally wherein the conjugate has an *in vitro* activity that is at least as high as an equivalent conjugate that was expressed in *E. coli.*

11. The conjugate for use according to any one of the preceding claims, wherein said treatment comprises:

    (a) intravenous administration of the conjugate; and/or
    (b) intramuscular administration of the conjugate.

12. The conjugate for use according to any one of the preceding claims, wherein said treatment comprises administration of the conjugate less than three times per week; optionally less than two times per week; optionally less than one time per week.

13. The conjugate for use according to any one of the preceding claims, wherein said disease treatable by L-asparagine depletion is a cancer; optionally wherein said cancer is selected from the group consisting of Acute Lymphoblastic Leukemia (ALL), non-Hodgkin's lymphoma, NK lymphoma, and pancreatic cancer; optionally wherein said cancer is ALL.

14. A composition comprising the conjugate for use according to any one of the preceding claims and a pharmaceutically acceptable excipient; optionally wherein the composition comprises one or more lyoprotectant(s); optionally wherein the lyoprotectant(s) are selected from the list consisting of trehalose and sucrose.

15. The composition for use according to claim 14, wherein the composition is:

    (a) in lyophilised form; or
    (b) in solubilised form.

Figure 1

R² = 0.9999

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RAU et al.** *Pediatr Blood Cancer.*, 2018, vol. 65, e26873
- **LANVERS et al.** *Analytical Biochemistry,* 2002, vol. 309 (1), 117-126
- **HENIKOFF ; HENIKOFF.** *Proc. Natl. Acad. Sci. USA,* 1992, vol. 89, 10915-10919
- **PATEL, V.J. et al.** *Journal of Proteome Research,* 2009, vol. 8, 3752-3759
- **JENSEN et al.** *Immunology,* 2018, vol. 154, 394-406